# EUROPEAN PATENT SPECIFICATION

(11) **EP 2 158 314 B1**
(45) Date of publication and mention of the grant of the patent: **18.12.2013**
(21) Application number: 09748006.5
(22) Date of filing: 31.03.2009
(51) Int. Cl.: C12N 15/06, C12N 15/10

(54) **A METHOD FOR IDENTIFYING A PORK CONTENT IN A FOOD**
VERFAHREN ZUR IDENTIFIKATION VON SCHWEINEBESTANDTEILEN IN EINEM NAHRUNGSMITTEL
PROCÉDÉ PERMETTANT D'IDENTIFIER UNE TENEUR EN PORC DANS UN ALIMENT

(30) Priority: 26.06.2008 MY 0802327
(43) Date of publication of application: 03.03.2010
(73) Proprietor: Universiti Putra Malaysia, Selangor Darul Ehsan 43400 (MY)
(72) Inventor: B. CHE MAN, Yaakob, 43400 Selangor Darul Ehsan (MY); MUSTAFA, Shuhaimi, 43400 Selangor Darul Ehsan (MY); KHALID, Farihah Liyana, 43400 Selangor Darul Ehsan (MY); AZMI , Aida Azrina, 43400 Selangor Darul Ehsan (MY); SAZILI , Awis Qurni, 43400 Selangor Darul Ehsan (MY); ABDUL RAHIM, Raha, 43400 Selangor Darul Ehsan (MY)
(74) Representative: Diaz Nunez, Joaquin
(86) International application number: PCT/MY2009/000047
(87) International publication number: WO 2009/157750

(56) References cited:
- EP-A1- 2 027 771
- EP-A2- 1 364 068
- WO-A1-2007/119066
- CN-A- 101 196 463
- JP-A- 2003 164 287
- AIDA A A ET AL: "Analysis of raw meats and fats of pigs using polymerase chain reaction for Halal authentication" MEAT SCIENCE, ELSEVIER SCIENCE, GB LNKD- DOI:10.1016/J.MEATSCI.2004.06.020, vol. 69, no. 1, 1 January 2005 (2005-01-01), pages 47-52, XP025283105 ISSN: 0309-1740 [retrieved on 2005-01-01]
- FAROUK, A.E. ET AL.: 'The use of a molecular technique for the detection of porcine ingredients in the Malaysian food market.' SAUDI MED J. vol. 27, no. 9, 2006, pages 1397 - 400
- CALVO, J.H. ET AL.: 'Technical note: A quick and more sensitive method to identify pork in processed and unprocessed food by PCR amplification of a new specific DNA fragment.' J ANIM SCI. vol. 79, no. 8, 2001, pages 2108 - 12, XP008128325
- RASTOGI, G. ET AL.: 'Species identification and authentication of tissues of animal origin using mitochondrial and nuclear markers.' MEAT SCIENCE. vol. 26, no. 4, 2007, pages 666 - 674, XP008128326
- LOPEZ-ANDREO, M. ET AL.: 'Identification and quantitation of species in complex DNA mixtures by real-time polymerase chain reaction.' ANAL BIOCHEM. vol. 339, no. 1, 2005, pages 73 - 82, XP004781302

## Description

### FIELD OF INVENTION

The invention relates to a method for designing primers for identification of food ingredients especially meat-based processed food. In particular, the method is to identify the presence of pork (*Sus scrofa*) in processed food for Halal authentication.

### BACKGROUND OF INVENTION

Food adulteration is a common issue worldwide. For instance, cheaper meats were used as a substitute for more expensive meats. Most frequently, pork meat has been used to substitute other meat types in food products. Therefore, the identification of animal species especially pork in food products is becoming an important issue to consumers. The implication of misleading the labeling of food can be much more important concerning the presence of potentially non-Halal food. For this reason, several methods have been developed to identify the species of origin of fresh meat and meat products. Numerous methods based on DNA analysis have been employed in the food industry to monitor adulterations of food products. Methods established for animal speciation are mostly lipid-,protein- and DNA-based. However, DNA-based methods are particularly more reliable as DNA is more stable under conditions associated with the high temperatures, pressures and chemical treatment used in food processing.

Species identification of animal tissues in meat products is an important issue to protect the consumer from illegal or undesirable adulteration; for economic, religious and health reasons. For this purpose, numerous analytical methods have been developed based on protein and DNA analysis. Among the DNA-based methods that are highly developed for species identification are species-specific conventional PCR and real-time PCR. Among the targeted gene fragments developed for pork species specific PCR are those derived from 12S rRNA ND5, Mitochondrial Displacement Loop (D-Loop) and Nuclear Melanocortin receptor 1 (MCIR).

Although method utilizing conventional PCR was proven to be successful, it requires a post-PCR manipulation that extends analysis time and handling hazardous chemical that may cause laboratory contamination. On the other hand, real-time PCR methods posses a great potential to replace the conventional PCR. This is mainly because real-time PCR methods are rapid, sensitive, specific, high degree of automation and target quantification (Heid *et al*,1996).

### SUMMARY OF THE INVENTION

The present invention relates to a method for identifying a pork content in a food, wherein the method includes the steps of extracting deoxyribonucleic asid (DNA) from a sample pork and designing a forward primer and a reverse primer based on ND5 mitochondrial gene of the pork by conducting a polymerase chain reaction (PCR) test on the forward and reverse primers characterized in that the sequence of the forward primer is SUS-FWD:5'-AGC TGC ACT ACA AGC AAT CC-3') and the sequence of the reverse primer is SUS-RVS:5'-ATG CGT TTG AGT GGG TTA GG-3'.

### BRIEF DESCRIPTION OF THE DRAWING

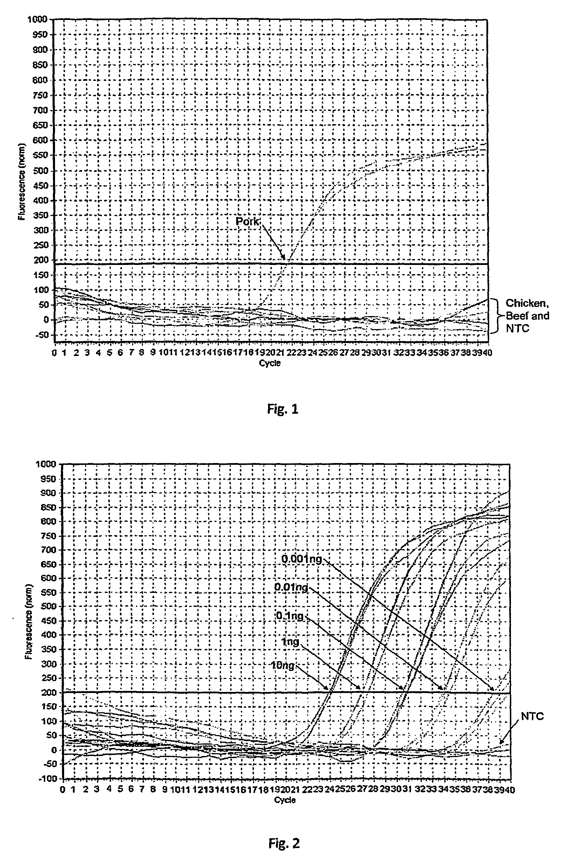

Fig. 1 shows the specificity test on pork primer designed against other meat species (beef and chicken).
Fig. 2 shows the sensitivity test of pork primer with 10-fold serial dilutions.

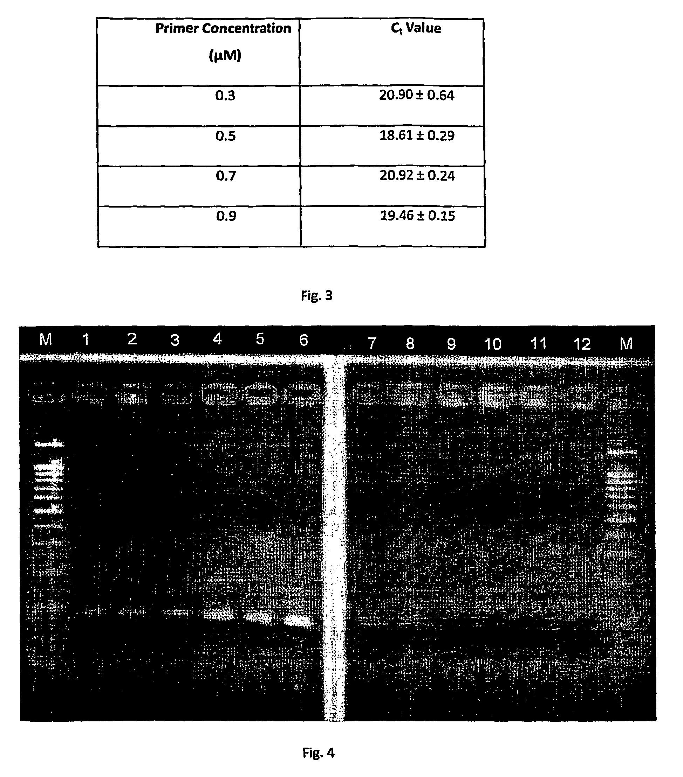

Fig. 3 shows optimization of primer concentration.
Fig. 4 shows the optimization of primer annealing temperature.

### DETAILED DESCRIPTION OF THE INVENTION

The invention describes the development and application of pork-specific real-time PCR assay for Halal authentication. Primers are designed to amplify an 89 bp amplicon of the pork ND5 mitochondrial (ND5) gene and were mismatched to commercial species of chicken and beef. The assay is highly sensitive and detected the presence of 0.001 ng of pork template DNA when assessed using dilutions of DNA in water. The primers set developed for Halal product verification are based on ND5 mitochondrial gene of pork and the sequence of the primers is as follows :
Forward primer (SUS-FWD: 5'-AGC TGC ACT ACA AGC AAT CC-3')
Reverse primer (SUS-RVS:5'-ATG CGT TTG AGT GGG TTA GG-3')

PCR conditions on detection of pork DNA is done by amplification in the Mastercycler ep (Eppendorf AG,Hamburg,Germany). Each reaction tube contains 20µl of reaction mixture which consists of 10 µl 2x Quantitect SYBR Green PCR Master Mix (Qiagen,Hilden,Germany), 1µl forward primer, 1 µl reverse primer, 3 µl dH20 and 5 µl DNA sample(20ng/ µl). The 3-step amplification cycle program is as follows: initial activation at 95°C for 15 min, denaturation at 94°C for 15s, annealing at 58°C for 30 s and extension at 72°C for 30 s. The initial activation step is to activate HotStarTaq DNA Polymerase present in the reaction mixture. The cycle is repeated 40 times and a melting curve analysis was performed to verify the specificity and identity of the amplified DNA.

### Samples

Pork, beef and chicken are the three species used in this study. The meat samples are purchased from a local wet market, Selangor Wholesale Market. They are stored at -20°C until used for DNA extraction.

### DNA Extraction

DNA from meat samples is extracted using DNeasy^{®} Blood and Tissue Kit (Qiagen,Hilden,Germany). 25mg of sample is weighed in a 1.5 ml microcentrifuge tube. 180 µl of Buffer ATL and 20 µl of proteinase K was added. The mixture is vortexed and then incubated overnight in a 56°C water bath for lysis. When samples is completely lysed, 4µl of RNase A (100mg/ml) was added, mixed and incubated at room temperature for 2 min. The mixture is vortexed before adding 200 µl Buffer AL and then vortexed again to mix thoroughly. Then, 200 µl ethanol (96-100%) is added and mixed by vortexing to yield a homogenous solution. The mixture is pipetted into the DNeasy Mini spin column set and centrifuged at 8000 rpm for 1 min. The flow-through and the collection tube are discarded. The DNeasy Mini spin column is then placed into a new 2 ml collection tube. 500 µl Buffer AW2 is added and centrifuged at 14,000 rpm for 3 min to ensure the column is dry and no ethanol carryover occur. The DNeasy Mini spin column is then placed into a new 1.5 ml microcentrifuge tube and 100 µl Buffer AF was added for elution. The tube is incubated for 1 min at room temperature and then centrifuged for 1 min at 8,000 rpm. The supernatant containing the extracted DNA is stored at 4°C before further use.

### Primer design

The Primer3 software (http://frodo.wi.mit.edulcgi-bin/primer3/primer3_www.cgi) is utilized for designing the primers set used in the real-time PCR. Sequences of the ND5 gene from pork (NC_000845), beef (NC_006853) and chicken (NC_001323) obtained from the NCBI GenBank database (hhtp://www.ncbi.nlm.nih.gov), are aligned and compared. One primers set (SUS-FWD:5'-AGC TGC ACT ACA AGC AAT CC-3' and SUS-RVS:5'-ATG CGT TTG AGT GGG TTA GG-3') was synthesized to specifically amplify an 89 bp fragment of the ND5 gene of pork.

### Real-time PCR analysis

Detection of pork DNA is done by amplification in the Mastercycler ep (Eppendorf AG, Hamburg, Germany). Each reaction tube contains 20µl of reaction mixture which consists of 10 µl 2x Quantitect SYBR Green PCR Master Mix (Qiagen, Hilden, Germany), 1 µl forward primer, 1 µl reverse primer, 3 µl dH₂O and 5 µl DNA sample. The 3-step amplification cycle program is as follows: initial activation at 95°C for 15 min, denaturation at 94°C for 15s, annealing at 58°C for 30s and extension at 72°C for 30s. The initial activation step is to activate HotStarTaq DNA Polymerase present in the reaction mixture. The cycle is repeated 40 times and a melting curve analysis was performed to verify the specificity and identity of the amplified DNA. Unless otherwise indicated, all reactions are carried out in triplicates.

## Claims

1. A method for identifying a pork content in a food, wherein the method includes the steps of :
(a) extracting deoxyribonucleic acid (DNA) from a sample pork
(b) designing a forward primer and a reverse primer based on ND5 mitochondrial gene of the pork; and
(c) conducting a polymerase chain reaction (PCR) test on the forward and reverse primers
**characterized in that**
the sequence of the forward primer is SUS-FWD:5'-AGC TGC ACT ACA AGC AAT CC-3') and the sequence of the reverse primer is SUS-RVS:5'-ATG CGT TTG AGT GGG TTA GG-3'.

2. The method as claimed in claim 1, wherein the concentration of the forward and reverse primers is between 0.3 to 0.9 µm.

3. The method as claimed in claim 1 wherein the reaction temperature is between 50-70°C.

4. A method for identifying a pork content in a food, wherein the method includes the steps of :
(a) designing a forward primer and a reverse primer based on ND5 mitochondrial gene of the pork; and
(b) conducting a polymerase chain reaction (PCR) test on the forward and reverse primers
**characterized in that**
the sequence of the forward primer is SUS-FWD:5'-AGC TGC ACT ACA AGC AAT CC-3') and the sequence of the reverse primer is SUS-RVS:5'-ATG CGT TTG AGT GGG TTA GG-3'.

5. The method as claimed in claim 4, wherein the concentration of the forward and reverse primers is between 0.3 to 0.9 µm.

6. The method as claimed in claim4 wherein the reaction temperature is between 50-70°C.

## Patentansprüche

1. Verfahren zur Ermittlung des Gehalts an Schweinefleisch in einem Lebensmittel, wobei das Verfahren folgende Schritte umfasst:
(a) Desoxyribonukleinsäure (DNA) aus einer Schweinefleischprobe extrahieren;
(b) einen Forward-Primer und einen Reverse-Primer entwerfen, basierend auf dem mitochondrialen ND5 Gen des Schweins; und
(c) einen Versuch einer Polymerase-Kettenreaktion (PCR) an den Forward- und Reverse-Primem durchführen;
**dadurch gekennzeichnet, dass**
die Sequenz des Forward-Primers SUS-FWD: 5'-AGC TGC ACT ACA TGC AAT CC-3') ist und die Sequenz des Reverse-Primers SUS-RVS: 5'-ATG CGT TTG AGT GGG TTA GG-3'.

2. Verfahren nach Anspruch 1, bei dem die Konzentration der Forward- und Reverse-Primer zwischen 0,3 und 0,9 µm liegt.

3. Verfahren nach Anspruch 1, bei dem die Reaktionstemperatur zwischen 50°C und 70 °C liegt.

4. Verfahren zur Ermittlung des Gehalts an Schweinefleisch in einem Lebensmittel, wobei das Verfahren folgende Schritte umfasst:
(a) einen Forward-Primer und einen Reverse-Primer entwerfen, basierend auf dem mitochondrialen ND5 Gen des Schweins; und
(b) einen Versuch einer Polymerase-Kettenreaktion (PCR) an den Forward- und Reverse-Primern durchführen;
**dadurch gekennzeichnet, dass**
die Sequenz des Forward-Primers SUS-FWD: 5'-AGC TGC ACT ACA AGC AAT CC-3') ist und die Sequenz des Reverse-Primers SUS-RVS: 5'-ATG CGT TTG AGT GGG TTA GG-3'.

5. Verfahren nach Anspruch 4, bei dem die Konzentration der Forward- und Reverse-Primer zwischen 0,3 und 0,9 µm liegt.

6. Verfahren nach Anspruch 4, bei dem die Reaktionstemperatur zwischen 50°C und 70 °C liegt.

## Revendications

1. Procédé identifiant un contenu porcin dans un aliment et incluant les phases suivantes:
(a) extraction d'acide désoxyribonucléigue (ADN) d'un échantillon de porc;
(b) conception d'un amorceur direct et d'un amorceur inverse sur la base du gène mitochondrial ND5 du porc; et
(c) réalisation d'un test de réaction en chaîne par polymérase PCR) sur les amorceurs directs et inverses ;
**caractérisé en ce que**:
la séquence de l'amorceur direct est de SUS-FWD: 5'-AGC TGC ACT AGA AGC AAT CC-3') et la séquence de l'amorceur inverse est de SUS-RVS: 5'-ATG CGT TTG AGT GGG TTA GG-3'.

2. Procédé, tel qu'indiqué dans la revendication 1, dans lequel la concentration des amorceurs directs et inverses est comprise entre 0,3 et 0,9 µm.

3. Procédé, tel qu'indiqué dans la revendication 1, dans lequel la température de réaction est comprise entre 50 °C et 70 °C.

4. Procédé identifiant un contenu porcin dans un aliment et incluant les phases suivantes:
(a) conception d'un amorceur direct et d'un amorceur inverse sur la base du gène mitochondrial ND5 du porc; et
(b) réalisation d'un test de réaction en chaîne par polymérase (PCR) sur les amorceurs directs et inverses ;
**caractérisé en ce que**:
la séquence de l'amorceur direct est de SUS-FWD: 5'-AGC TGC ACT ACA TGC AAT CC-3') et la séquence de l'amorceur inverse est de SUS-RVS: 5'-ATG CGT TTG AGT GGG TTA GG-3'.

5. Procédé, tel qu'indiqué dans la revendication 4, dans lequel la concentration des amorceurs directs et inverses est comprise entre 0,3 et 0,9 µm.

6. Procédé, tel qu'indiqué dans la revendication 4, dans lequel la température de réaction est comprise entre 50°C et 70 °C.
